## Europäisches Patentamt

## European Patent Office

## Office européen des brevets

(19)

(11) Veröffentlichungsnummer: **0 130 595**
**B1**

(12) # EUROPÄISCHE PATENTSCHRIFT

(45) Veröffentlichungstag der Patentschrift:
08.03.89

(21) Anmeldenummer: 84107572.4

(22) Anmeldetag: 29.06.84

(51) Int. Cl.⁴: **B 01 J 8/06** //
C07C51/265, C07C51/31

(54) Reaktor zur Durchführung von stark exothermen und endothermen katalytischen Prozessen.

(30) Priorität: 01.07.83 BG 61567/83

(43) Veröffentlichungstag der Anmeldung:
09.01.85 Patentblatt 85/2

(45) Bekanntmachung des Hinweises auf die Patenterteilung:
08.03.89 Patentblatt 89/10

(84) Benannte Vertragsstaaten:
AT BE CH DE FR GB IT LI

(56) Entgegenhaltungen:
DE-A-2 016 614
DE-A-2 222 958
DE-A-2 830 765
FR-A-1 479 895
FR-A-2 016 264
US-A-2 306 011

(73) Patentinhaber: STOPANSKI CHIMITCHESKI KOMBINAT "GAVRIL GENOV", Boul. H. Smirnenski 21, Russe (BG)

(72) Erfinder: Nikolov, Valentin Assenov, 17, Borimetchka Str., Russe (BG)
Erfinder: Klissurski, Dimiter Georgiev, 23, Dospat Str., Sofia (BG)
Erfinder: Jurov, Boyan Manolov, Komplex Sdravez Block "Jelyo Voyvoda", Russe (BG)

(74) Vertreter: Finck, Dieter, Patentanwälte v. Füner, Ebbinghaus, Finck Mariahilfplatz 2 & 3, D-8000 München 90 (DE)

EP 0 130 595 B1

**Beschreibung**

Die Erfindung betrifft einen Reaktor zur Durchführung von stark wärmegetönten katalytischen Prozessen, mit einem Mantel, in dem senkrecht zur Achse des Reaktors Rohre angeordnet sind, die von einem Wärmeträger durchflossen sind auf auf deren Außenflächen ein Katalysator aufgetragen ist, wobei der Mantel mit einer oberen Eingabeöffnung für Rohstoffe und mit einer Produkt-Abgabeöffnung versehen ist.

Ein solcher, aus der DE-A-2 222 958 bekannter Reaktor dient zur Durchführung von stark exothermen katalytischen Prozessen. Der Reaktor hat einen Reaktionsabschnitt, der den Hauptabschnitt des Reaktors bildet. Dieser Reaktionsabschnitt besteht aus einer Anzahl von unmittelbar aufeinanderfolgenden Sektionen. Jede Sektion ist aus einem Rahmen zusammengesetzt, der mit im wesentlichen horizontal angeordneten Rohren für ein Kühlmittel versehen ist. Die äußere Oberfläche der Rohre ist mit einem Katalysatorträger mit einem darauf aufgebrachten Festbett-Katalysator bedeckt.

Aufgrund des standardisierten Aufbaus der Sektionen ist ein optimales Temperaturprofil in dem bekannten Reaktor nur schwer einzuhalten. Bei unzureichender Wärmeabfuhr durch den Wärmeträger können sogar Heißstellen entstehen, die für den Reaktor eine Gefahr darstellen.

Aus der DE-A-2 830 765 ist ferner bekannt, das Innere eines Festbett-Reaktors mit vertikalen Rohren in wenigstens zwei Aufgabezonen für ein Wärmeableitungsmedium zu unterteilen. Dabei läuft die Reaktion innerhalb der Rohre ab. Die Rohre werden außen von dem Wärmeträger umspült, und zwar ausschließlich im Bereich der einzigen vorhandenen großen Reaktionszone. Eine Aufteilung in Wärmeaustauchbereiche und Reaktionsbereiche ist nicht vorgesehen.

Die der Erfindung zugrunde liegende Aufgabe besteht nun darin, den Reaktor der gattungsgemäßen Art so auszubilden, daß unter Vermeidung der Bildung von Heißstellen beliebige Temperaturprofile genau eingehalten werden können.

Diese Aufgabe wird bei dem Reaktor der gattungsgemäßen Art dadurch gelöst, daß die Rohre aufeinanderfolgend Gruppen von Wärmeaustauchbereichen und Reaktionsbereichen bilden, wobei nur auf den Rohren in den Reaktionsbereichen Katalysator aufgetragen ist, und daß der Mantel des Reaktors in Höhe der Reaktionsbereiche mit seitlichen Stutzen zur Einführung von Rohstoffen versehen ist.

Diese Ausgestaltung des erfindungsgemäßen Reaktors ermöglicht durch die abwechselnde Aufeinanderfolge von Bereichen, in denen nur ein Wärmeaustausch stattfindet, und von Bereichen, in denen zusätzlich die katalystische Reaktion abläuft, daß das für die jeweilige Reaktion optimale Temperaturprofil eingehalten werden kann, wobei die Möglichkeit der zusätzlichen Einführung der Rohstoffe durch die seitlichen Stützen zur genauen Temperatureinstellung beiträgt.

Zweckmäßigerweise befinden sich zu beiden Seiten jedes Reaktionsbereichs Wärmeaustauschbereiche. Dabei befindet sich jeweils zwischen Reaktionsbereichen wenigstens ein Wärmeaustauschbereich.

In verschiedenen Reaktionsbereichen können verschiedene Katalysatoren verwendet werden.

Vorteilhafterweise sind die Reaktionsbereiche und die Wärmeaustauschbereiche unter einem Winkel zueinander angeordnet. Anhand der Zeichnungen wird die Erfindung beispielsweise näher erläutert. Es zeigt:

Fig. 1 im Längsschnitt eine erste Ausführungsform eines Reaktors und

Fig. 2 im Längsschnitt einen Reaktor, bei welchem die Bereiche unter einem Winkel angeordnet sind und zwischen den Reaktionsbereichen jeweils ein Wärmeaustauschbereich liegt.

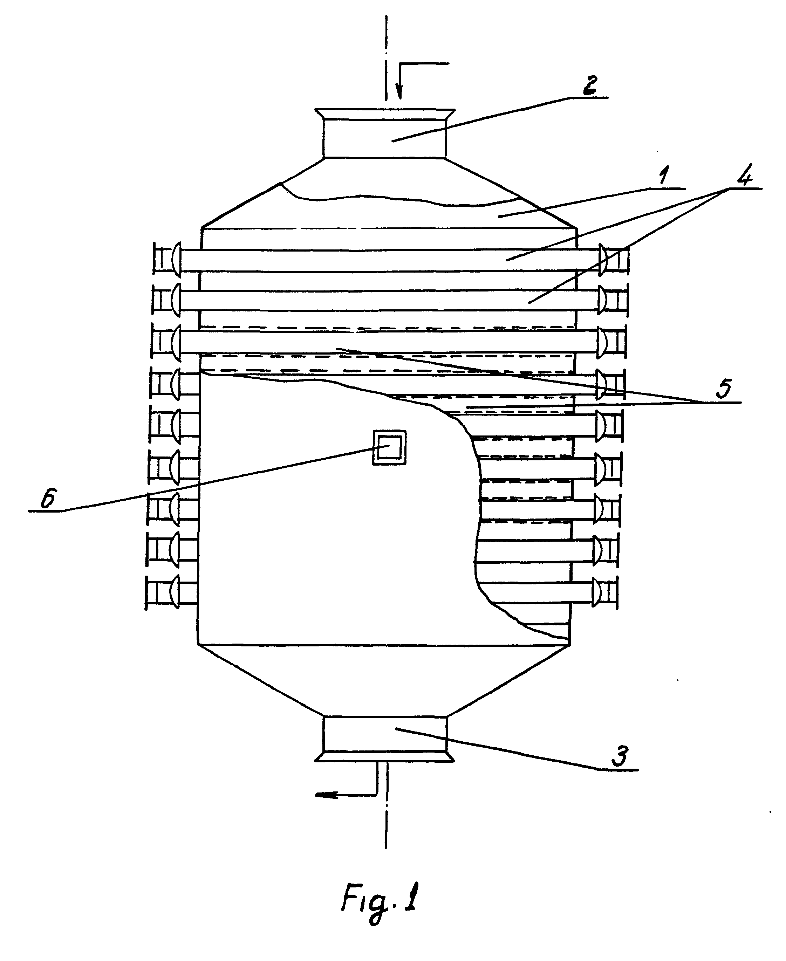

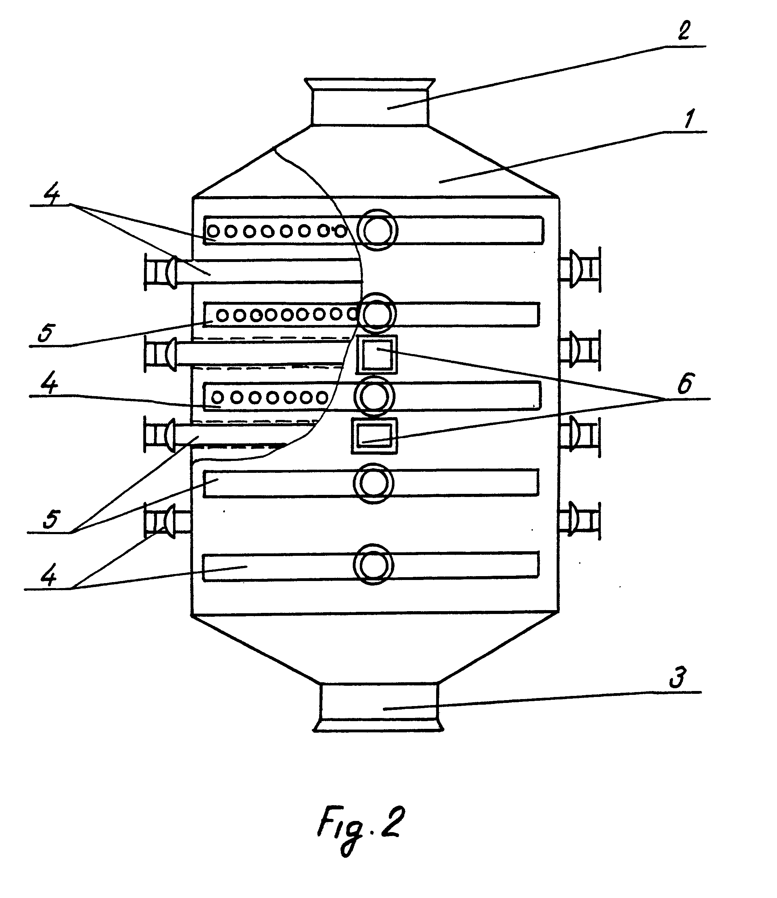

Der Reaktor zur Durchführung von stark exothermen und endothermen katalytischen Prozessen besteht aus einem Mantel 1, in dessen Ober- und Unterteil Rohre angeordnet sind, die in Wärmeaustauschbereiche 4 gruppiert sind, zwischen denen Rohre angeordnet sind, auf deren Außenseite ein Katalysator aufgetragen ist. Diese Rohre sind ihrerseits in Reaktionsbereiche 5 gruppiert. Die Reaktionsbereiche 5 und die Wärmeaustauschbereiche 4 sind senkrecht zur Achse des Reaktors angeordnet. Der Katalysator kann dieselbe oder eine unterschiedliche Zusammensetzung haben. Die Wärmeaustauschbereiche 4 und die Reaktionsbereiche 5 können glatt sein oder mit Rippen versehen sein sowie unter einem Winkel angeordnet sein (Fig. 2). Der Mantel 1 ist mit einer oberen Eingabeöffnung 2 und einem seitlichen Stutzen 6 (Fig. 1) zur Zuführung der Ausgangsstoffe oder mit seitlichen Stutzen 6 (Fig. 2) zur Zuführung der Rohstoffe sowie mit einer Produktabgabeöffnung 3 zur Abführung der Reaktionsprodukte aus dem Reaktor versehen.

Der Reaktor arbeitet folgendermaßen:

Die Rohstoffe werden durch die obere Eingabeöffnung 2 und die seitlichen Stutzen 6 zugeführt. Sie gehen durch die Wärmeaustauschbereiche 4 hindurch und werden auf die notwendige Temperatur erwärmt. Danach gehen sie durch die Reaktionsbereiche 5 hindurch, wo die katalytische Reaktion abläuft. Die optimale Temperatur des Reaktors wird durch den Wärmeträger erreicht, der im Inneren der Wärmeaustauschbereiche 4 und der Reaktionsbereiche 5 zirkuliert, sowie durch die Menge der durch die obere Eingabeöffnung 2 und die seitlichen Stutzen 6 zugeführten Rohstoffe. Wenn die Reaktionsprodukte durch die Reaktionsbereiche 5 hindurchgegangen sind, werden sie auf die notwendige Temperatur abgekühlt. Sie gehen durch die Wärmeaustauschbereiche 4 hindurch und verlassen den Reaktor durch die Produktabgabeöffnung 3.

## Patentansprüche

1. Reaktor zur Durchführung von stark wärmegetönten katalytischen Prozessen, mit einem Mantel, in dem senkrecht zur Achse des Reaktors Rohre angeordnet sind, die von einem Wärmeträger durchflossen sind und auf deren Außenflächen ein Katalysator aufgetragen ist, wobei der Mantel mit einer oberen Eingabeöffnung für Rohstoffe und mit einer Produkt-Abgabeöffnung versehen ist, dadurch gekennzeichnet, daß

- die Rohre aufeinanderfolgend Gruppen von Wärmeaustauschbereichen (4) und Reaktionsbereichen (5) bilden, wobei nur auf den Rohren in den Reaktionsbereichen (5) Katalysator aufgetragen ist, und daß

- der Mantel (1) des Reaktors in Höhe der Reaktionsbereiche (5) mit seitlichen Stutzen (6) zur Einführung von Rohstoffen versehen ist.

2. Reaktor nach Anspruch 1, dadurch gekennzeichnet, daß sich zu beiden Seiten jedes Reaktionsbereiches (5) Wärmeaustauschbereiche (4) befinden.

3. Reaktor nach Anspruch 2, dadurch gekennzeichnet, daß sich jeweils zwischen Reaktionsbereichen (5) wenigstens ein Wärmeaustauschbereich (4) befindet.

4. Reaktor nach Anspruch 1, dadurch gekennzeichnet, daß in verschiedenen Reaktionsbereichen (5) verschiedene Katalysatoren verwendet sind.

5. Reaktor nach Anspruch 1, dadurch gekennzeichnet, daß die Reaktionsbereiche (5) und die Wärmeaustauschbereiche (4) unter einem Winkel zueinander angeordnet sind.

## Claims

1. Reactor for carrying out strongly thermally profiled catalytic processes with a casing in which tubes are arranged at right angles to the axis of the reactor, through which tubes on whose outer surfaces a catalyst is supported, flows a heat carrier, with the casing being provided with an upper input opening for raw materials and with a product delivery opening, characterised in that

- the tubes form sequentially groups of heat exchanging regions (4) and reaction regions (5), with catalyst being supported only on the tubes in the reaction regions (5), and that

- the casing (1) of the reactor is provided at the height of the reaction regions (5) with lateral connections for introduction of raw materials.

2. Reactor according to claim 1, characterised in that heat exchanging regions (4) are located on both sides of each reaction region (5).

3. Reactor according to claim 2, characterized in that at least one heat exchanging region (4) is located in each case between reaction regions (5).

4. Reactor according to claim 1, characterised in that different catalysts are used in different reaction regions (5).

5. Reactor according to claim 1, characterized in that the reaction regions (5) and the heat exchanging regions (4) are arranged at an angle to one another.

## Revendications

1. Réacteur pour la mise en oeuvre de procédés catalytiques impliquant une forte chaleur de réaction, comportant une enveloppe dans laquelle sont disposés, perpendiculairement à l'axe du réacteur, des tubes qui sont parcourus par un fluide caloporteur et sur les surfaces extérieures desquels est disposé un catalyseur, l'enveloppe étant pourvue d'une ouverture supérieure d'admission pour les matières premières et d'une ouverture d'évacuation de produit, caractérisé en ce que les tubes forment des groupes successifs de zones d'échange thermique (4) et de zones réactionnelles (5), le catalyseur n'étant déposé que sur les tubes situés dans les zones réactionnelles (5), et en ce que l'enveloppe (1) du réacteur est pourvue, à la hauteur des zones réactionnelles (5), de tubulures latérales (6) pour l'introduction de matières premières.

2. Réacteur selon la revendication 1, caractérisé en ce que les zones d'échange thermique (4) sont situées des deux côtés de chaque zone réactionnelle (5).

3. Réacteur selon la revendication 2, caractérisé en ce qu'au moins une zone d'échange thermique (4) est située respectivement entre des zones réactionnelles (5).

4. Réacteur selon la revendication 1, caractérisé en ce que différents catalyseurs sont utilisés dans différentes zones réactionnelles (5).

5. Réacteur selon la revendication 1, caractérisé en ce que les zones réactionnelles (5) et les zones d'échange thermique (4) sont disposées de manière à former un angle entre elles.

Fig. 1

Fig.2